# EUROPEAN PATENT SPECIFICATION

(11) **EP 1 097 174 B1**
(45) Date of publication and mention of the grant of the patent: **08.06.2005**
(21) Application number: 99930930.5
(22) Date of filing: 09.07.1999
(51) Int. Cl.: C07K 16/34, C07K 16/36, G01N 33/86, G01N 33/96, C12N 5/18

(54) **LUPUS ANTICOAGULANT ANTIBODY TO F1 REGION OF PROTHROMBIN**
LUPUS ANTIKOAGULANT-ANTIKÖRPER GEGEN DIE F1 REGION VON PROTHROMBIN
ANTICORPS ANTI-LUPUS ANTICOAGULANTS DIRIGES CONTRE LA REGION F1 DE LA PROTHROMBINE

(30) Priority: 10.07.1998 AU PP460398
(43) Date of publication of application: 09.05.2001
(73) Proprietor: Gradipore Limited, North Ryde, NSW 2072 (AU); Dade Behring Marburg GmbH, 35001 Marburg (DE)
(72) Inventor: EXNER, Thomas, Gordon, NSW 2072 (AU); KRAUS, Michael, A-1221 Vienna (AT)
(74) Representative: Daniels, Jeffrey Nicholas
(86) International application number: PCT/AU1999/000556
(87) International publication number: WO 2000/002925

(56) References cited:
- WO-A-91/06861
- WO-A-91/17177
- ARNOUT J ET AL: "Monoclonal antibodies against beta-2-glycoprotein I: Use as reference material for lupus anticoagulant tests." THROMBOSIS AND HAEMOSTASIS, vol. 79, no. 5, May 1998 (1998-05), pages 955-958, XP001071052 ISSN: 0340-6245
- PERMPIKUL PARICHART ET AL: "Functional and binding studies of the roles of prothrombin and beta-2-glycoprotein I in the expression of lupus anticoagulant activity." BLOOD, vol. 83, no. 10, 1994, pages 2878-2892, XP001079099 ISSN: 0006-4971
- T. EXNER ET AL: 'A Monoclonal Antibody against Prothrombin Fragment 1 Behaves Like a Lupus Anticoagulant' THROMBOSIS AND HAEMOSTASIS vol. 81(3), March 1999, pages 470 - 471
- C.L. CASIPIT ET AL: 'Improving the Binding Affinity of an Antibody Using Molecular Modeling and Site-Directed Mutagenesis' PROTEIN SCIENCE vol. 7, August 1998, pages 1671 - 1680
- M.J. HURSTING ET AL: 'Monoclonal Antibodies Specific for Prothrombin Fragment 1.2 and Their Use in a Quantitative Enzyme-Linked Immunosorbent Assay' CLINICAL CHEMISTRY vol. 39, no. 4, 1993, pages 583 - 591
- H. PELZER ET AL: 'Determination of Human Prothrombin Activation Fragment 1+2 in Plasma with an Antibody against a Synthetic Peptide' THROMBOSIS AND HAEMOSTASIS vol. 65(2), 1991, pages 153 - 159
- R.A. FLECK ET AL: 'Anti-Prothrombin Antibodies and the Lupus Anticoagulant' BLOOD vol. 72, no. 2, 1988, pages 512 - 519

## Description

### Technical Field

The present invention relates to reagents applicable for use in blood coagulation assays, particularly mimics of lupus anticoagulants.

### Background Art

Lupus anticoagulants (LA) are regarded as phospholipid-interfering antibodies, somehow related to anticardiolipin antibodies detectable in many patients with systemic lupus erythematosus (SLE) and anti-phospholipid syndrome [1]. The LA are not directed at phospholipids *per se*, but rather at epitopes of protein complexes which contain specific phospholipids. In recent years there has been increasing evidence that these epitopes are contained on either beta-2 glycoprotein 1 or prothrombin.

The subtype of LA acting through beta-2-glycoprotein 1 enhance the anticoagulant effect of this normally mild coagulation inhibitor and may increase its affinity for phospholipid. These LA are associated with phospholipid liposomes and may be detected by solid phase immunoassays. This class of LA is believed to be more readily detectable with the dilute Russell's viper venom time (DRVVT) test whereas those LA that function through prothrombin are said to have more effect on the kaolin clotting time (KCT) [2].

The prothrombin-dependent LA do not sediment with phospholipid liposomes and may not pose such a significant prothrombotic risk as LA acting through beta-2-glycoprotein 1. Antibodies against prothrombin have been reported in many cases of LA, however, the association between prothrombin and these antibodies cannot usually be demonstrated *in vitro* [3]. In severe cases, low levels of prothrombin develop because complexes formed *in vivo* are cleared from circulation. Such patients have an increased risk of bleeding and some studies have suggested that plasmas containing LA often contain antibodies recognising the F1.2 part of the prothrombin molecule [4]. Other reports have shown that enzyme linked immunosorbant assays (EUSAs) for F1.2 are not particularly sensitive to thrombotic risk in patients with lupus anticoagulants [5].

A variety of artificial standards for LA have been proposed for laboratory use. These have included phospholipases, annexins, polymyxin, quaternary ammonium detergents and monoclonal antibodies against beta-2-glycoprotein 1 [6]. None of these have been found to affect various clotting tests in the same pattern as typical LA. Although such agents show correction on addition of phospholipid similar to true LA, all except for monoclonal antibodies against beta-2-glycoprotein, the agents also show "correction" on mixing with normal plasma.

The present inventor has now produced antibodies that act similarly to LA found in patients with clotting abnormalities. These antibodies in plasma act similarly to LA in not being corrected by the addition of normal plasma in blood clotting tests. The antibodies' actions *in vitro* are reversed by the addition of additional phospholipids to clotting tests, which is also observed with LA [7]. The present invention also serves as a model for other potential interferences of autoimmune antibodies on activation peptides, in particular those from components of the protein C pathway.

### Disclosure of Invention

In a first aspect, the present invention consists in an isolated antibody directed against the F1 region of prothrombin, which antibody mimics the effect of a lupus anticoagulant (LA) *in vitro*. Said antibody is capable of binding F1.2 region of prothrombin but is not capable of binding F2 region of prothrombin.

Preferably, the antibody is a monoclonal antibody.

In a preferred embodiment, the monoclonal antibody is obtained from a mouse following immunisation with human prothrombin, preferably N-terminal peptides derived from human prothrombin. In obtaining suitable monoclonal antibodies, it is important to select clones producing antibodies directed to the F1 region of prothrombin. Suitable clones are usually selected by the ability of the antibody to bind F1.2 but not F2 region of prothrombin. Screening of clones is usually carried out using F1.2 coated wells of microtitre plates and testing for antibodies which bind thereto.

It would be expected by persons skilled in the art from the present disclosure and teaching that other suitable antibodies can be generated which act similarly to the antibody examples described herein.

Preferably, the monoclonal antibody is selected from 195/097a, 195/016, or 195/0155. More preferably, the antibody is 195/097a.

The preferred antibody according to the first aspect of the present invention (195/097a) will be available commercially or by request from the applicant Gradipore Limited at PO Box 1865, Macquarie Centre, New South Wales, 2113 Australia.

The antibodies can be generated using standard monoclonal antibody production techniques following appropriate immunisation of an animal, preferably a mouse.

In a second aspect, the present invention consists in use of an isolated antibody according to the first aspect of the present invention in an *in vitro* blood coagulation assay. Preferably, the assay is an activated partial thromboplastin time (APTT) test using a reagent sensitive to LA. Other screening tests are also suitable and include kaolin clotting time (KCT), tissue thromboplastin inhibition test (TTTT), dilute Russell's viper venom time (DRVVT), Taipan venom, and Textarin test.

The antibodies according to the present invention are particularly suitable as mimics of naturally occurring LA.

The present inventors have found that antibody amounts of about 0.01 to 10 µmol are suitable for many common blood clotting tests. An amount of up to about 0.7 µmol has been found to be maximally effective in some tests. This amount is approximately half the level of prothrombin in normal plasma. It will be appreciated, however, that the amount of antibody will vary depending on the test and particular use.

In a third aspect, the present invention consists in a blood clotting test standard, the standard comprising plasma treated or mixed with the antibody according to the first aspect of the present invention.

The standard is useful as a defined strength of lupus anticoagulant for control in clotting tests. The standard can be prepared by "spiking" normal human plasma with a defined amount of antibody and used to define the sensitivity of various clotting tests to LA.

The standard is particularly suitable for tests including activated partial thromboplastin time (APTT) test, kaolin clotting time (KCT), tissue thromboplastin inhibition test (TTTT), dilute Russell's viper venom time (DRVVT), Taipaan venom and Textarin test.

In a fourth aspect, the present invention consists in a method of obtaining an antibody according to the first aspect of the present invention, the method comprising:
(a) immunising an animal with prothrombin or a N-terminal peptide derived from human prothrombin;
(b) testing for the production of an antibody in the animal which is capable of binding F1.2 region of prothrombin but not capable of binding F2 region of prothrombin; and
(c) isolating the antibody.

Preferably the antibody is a monoclonal antibody.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

In order that the present invention may be more clearly understood, preferred forms will be described with reference to the following examples and drawings.

### Brief Description of Drawings

Figure 1 shows the effect of different monoclonal antibodies on an APTT test.
Figure 2 shows the effect of monoclonal antibodies against F1 on various blood clotting tests used for diagnosing LA.
Figure 3 shows a comparison of monoclonal antibodies with LA on an APTT test.

### Modes for Carrying Out the Invention

### METHODS

### Monoclonal Antibody Production

Monoclonal antibodies were produced by standard techniques [9] after immunising mice with whole prothrombin or peptides derived from human prothrombin.

### APTT Assay

Standard assay techniques were used [10, 11].

### RESULTS

The monoclonal antibodies of importance were generated by immunising mice with whole human prothrombin and then selecting clones which specifically bound to the F1 region. Other antibodies were generated by immunising mice with the C-terminal peptide of F1+2 fragment (Sequence: Cys-Gly-Ser-Asp-Arg-Ala-Ile-Glu-Gly-Arg; SEQ ID No.: 1) attached to ovalbumin. The amino acid sequence has been used previously to generate anti-F1+2 antibodies [8]. Surprisingly, it has been found by the present inventor that antibodies generated had lupus anticoagulant-like activity, a property which until now has been addressed to proteins binding to phospholipids. Interestingly, F1+2 is the inactive part of prothrombin after activation by prothrombin activators including the factor Xa/Va complex.

The monoclonal antibodies were generated in order to replace monospecific polyclonal antibodies (rabbit) which are not easy to produce by standard monoclonal techniques and which have very low activity in clotting tests.

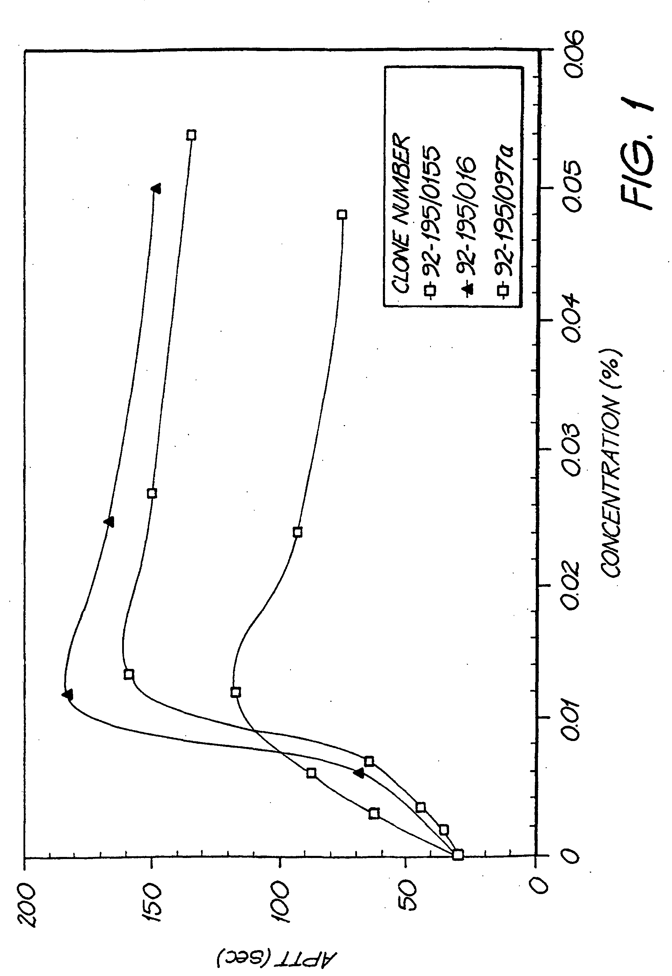

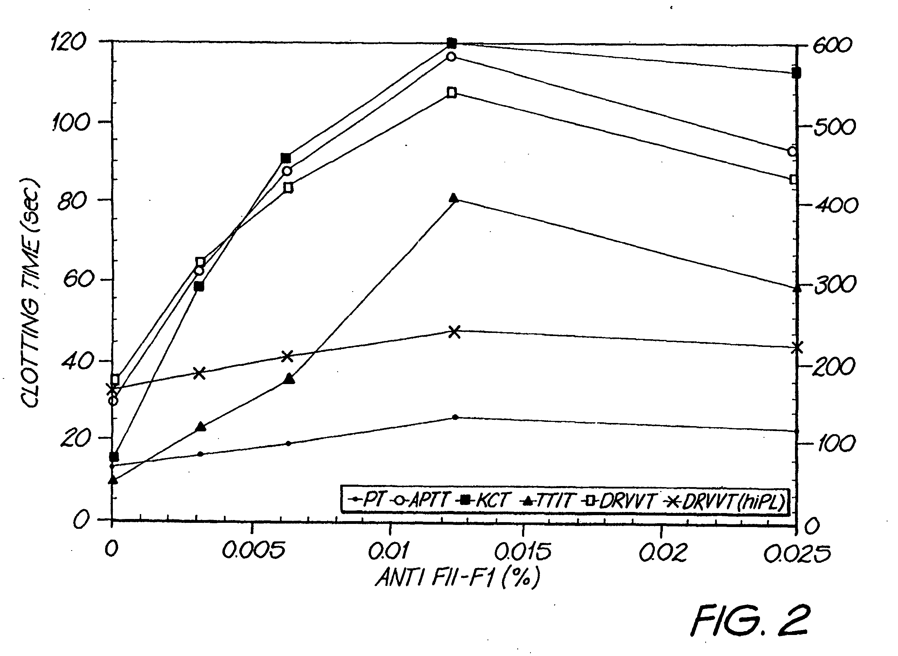

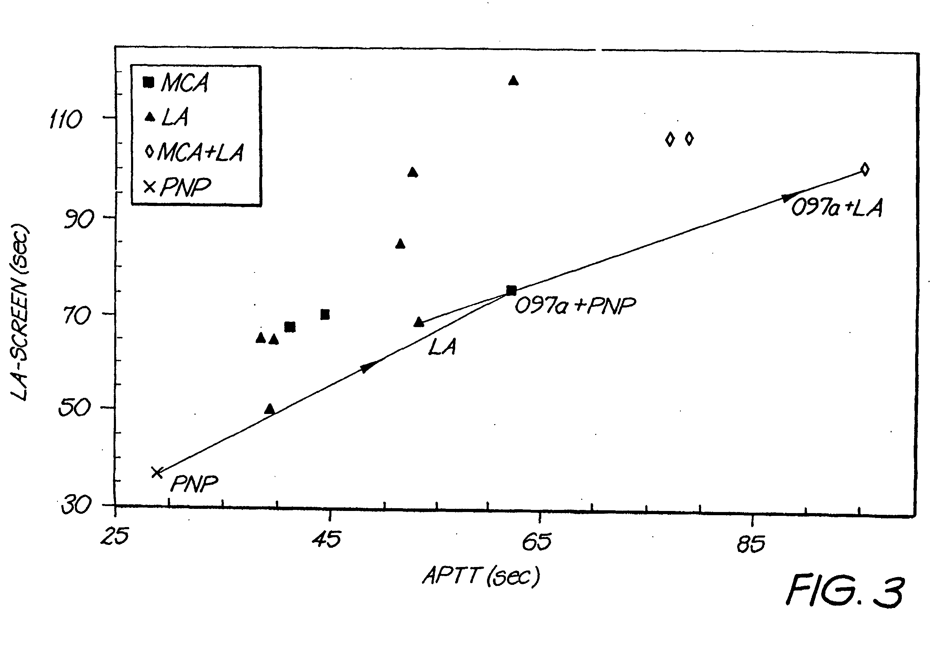

Monoclonal antibodies directed against the F1 and F1+2 parts of prothrombin were found to prolong all the clotting tests normally used for detecting lupus anticoagulants(LA) such as the APTT (see Figure 1), KCT, DRVVT, and TTIT. The strength of their effect on various clotting tests was in similar proportion as the sensitivity of these tests for typical LA (Figures 2 and 3). Phospholipids used for reversing the effect of LA in the DRVVT also reversed the anticoagulant effect of these antibodies. This is apparent from Figure 2 where plasma containing the anti-FII-F1 monoclonal gave prolonged results with DRVVT reagent but not with the DRVVT (high phospholipid). Thus such MCA against F1 and F1+2 fulfil the current criteria for LA and might be useful as known standards of LA potency. The functional similarity of the antibodies to LA strongly suggests that the majority of LA may also be directed at complex epitopes involving the F1 or F1+2 regions of prothrombin.

One of the monoclonal antibodies (92-195/097a, Figure 1) showed a typical lupus "cofactor" mixing pattern suggesting that subtle differences in F1 epitope may account for this phenomenon without any need for a separate cofactor.

### REFERENCES

1. Brandt JT. Triplett DA, Alving B, Scharrer I. Thombosis and Haemostasis 1995; 74; 1185-190.
2. Galli M. Finazzi G, Bevers EM, Barbui T. Blood. 1995; 86; 617-623.
3. Rao LVM, Huang AD, Rapaport SI. Thrombosis and Haemostasis. 1997; 73; 668-674.
4. Malia RG, Brookfield C, Bulman L, Greaves M. Thrombosis and Haemostasis. 1997; 78/2, 170 (OC-689).
5. Horbach DA, van Oort E, Donders RCJM, Derksen RHWM, de Groot PG, Thrombosis and Haemostasis. 1996; 76; 916-924.
6. Arnout J, Vanrusselt M, Wittevrongel C, Vermylen J. Thrombosis and Haemostasis. 1998; 79; 955-958.
7. Exner T Kraus M. Thrombosis and Haemostasis. 1999; 81; 470-471.
8. Husting MJ. *et al*. Clinical Chemistry. 1993; 89,583-591.
9. Galfé G, Milstein C. Preparation of monoclonal antibodies: Strategies and procedures. Methods Enzymol. 1981; 73; 3-46
10. Proctor RR, Rapaport SI. The partial thromboplastin time with kaolin. Am. J. Clin. Pathol. 1961; 36; 212-218.
11. Exner T. Diagnostic methodologies for circulating anticoagulants. Thrombosis and Haemostasis. 1985; 74; 338-344

## Claims

1. An isolated antibody for use in an *in vitro* blood clotting assay, the antibody
**characterised by**:
being directed against the F1 region of prothrombin;
being capable of binding F1.2 region of prothrombin but not capable of binding F2 region of prothrombin; and
being able to mimic the effect of a lupus anticoagulant (LA) in an *in vitro* blood clotting assay.

2. The antibody according to claim 1 being a monoclonal antibody.

3. The antibody according to claim 2 obtained from a mouse following immunisation with human prothrombin and selected for its ability to mimic the effect of a lupus anticoagulant (LA) in an *in vitro* blood clotting assay.

4. The antibody according to claim 3 wherein the mouse is immunised with a N-terminal peptide derived from human prothrombin.

5. The antibody according to any one of claims 1 to 4wherein the blood clotting assay is selected from the group consisting of activated partial thromboplastin time (APTT) test, kaolin clotting time (KCT), tissue thromboplastin inhibition test (TTIT), dilute Russell's viper venom time (DRVVT), Taipan venom, and Textarin test.

6. Use of an isolated antibody according to any one of claims 1 to 5 in an *in vitro* blood clotting assay.

7. The use according to claim 6 wherein the blood clotting assay is selected from the group consisting of activated partial thromboplastin time (APTT) test, kaolin clotting time (KCT), tissue thromboplastin inhibition test (TTIT), dilute Russell's viper venom time (DRVVT), Taipan venom, and Textarin test.

8. The use according to claim 6 or 7 wherein the amount of antibody is from 0.01 to 10 µmol.

9. The use according to claim 8 wherein the amount of antibody is up to 0.7 µmol.

10. A blood clotting test standard comprising normal human plasma treated or mixed with the antibody according to any one of claims 1 to 5.

11. The test standard according to claim 10 for use in blood clotting tests selected from the group consisting of activated partial thromboplastin time (APTT) teset, kaolin clotting time (KCT), tissue thromboplastin inhibition test (TTIT), dilute Russell's viper venom time (DRVVT), Taipan venom, and Textarin test.

12. A method of obtaining an antibody directed against the F1 region of prothrombin which mimics the effect of a lupus anticoagulant (LA) in an *in vitro* blood clotting test, the method comprising:
immunising a non-human animal with prothrombin or a N-terminal peptide derived from human prothrombin;
testing for the production of an antibody by the animal which is capable of binding F1.2 region of prothrombin but not capable of binding F2 region of prothrombin and which antibody mimics the effect of a lupus anticoagulant (LA) in an *in vitro* blood clotting test; and
isolating the antibody.

## Patentansprüche

1. isolierter Antikörper für die Verwendung in einem *In-Vitro*-Blutgerinnungstest, wobei der Antikörper **dadurch gekennzeichnet ist, dass** er
gegen den F1-Bereich des Prothrombins gerichtet ist,
den F1.2-Bereich des Prothrombins binden kann, jedoch den F2-Bereich des Prothrombins nicht binden kann, und
die Wirkung eines Lupus-Anticoagulans (LA) in einem *In-Vitro* -Blutgerinnungstest nachahmen kann.

2. Antikörper nach Anspruch 1, bei dem es sich um einen monoklonalen Antikörper handelt.

3. Antikörper nach Anspruch 2, erhalten aus einer Maus nach Immunisierung mit menschlichem Prothrombin und ausgewählt auf seine Fähigkeit, die Wirkung eines Lupus-Anticoagulans (LA) in einem *In*-*Vitro-*Blutgerinnungstest nachzuahmen.

4. Antikörper nach Anspruch 3, worin die Maus mit einem N-terminalen Peptid immunisiert wird, das von menschlichem Prothrombin abgeleitet ist.

5. Antikörper nach einem der Ansprüche 1 bis 4, worin der Blutgerinnungstest ausgewählt ist aus der Gruppe, bestehend aus dem Test auf die aktivierte partielle Thromboplastinzeit (APTT), der Kaolin-Gerinnungszeit (KCT), dem Gewebsthromboplastin-Inhibitionstest (TTIT), der Zeit von verdünntem Russell-Schlangengift (dRVVT = dilute Russells's viper venom time), Taipan-Gift und dem Textarintest.

6. Verwendung eines isolierten Antikörpers nach einem der Ansprüche 1 bis 5 in einem *In-Vitro*-Blutgerinnungstest.

7. Verwendung nach Anspruch 6, worin der Blutgerinnungstest ausgewählt ist aus der Gruppe, bestehend aus dem Test auf die aktivierte partielle Thromboplastinzeit (APTT), der Kaolin-Gerinnungszeit (KCT), dem Gewebsthromboplastin-Inhibitionstest (TTIT), der Zeit von verdünntem Russell-Schlangengift (dRVVT = dilute Russells's viper venom time), Taipan-Gift und dem Textarintest.

8. Verwendung nach Anspruch 6 oder 7, worin die Menge an Antikörper von 0,01 bis 10 µmol beträgt.

9. Verwendung nach Anspruch 8, worin die Menge an Antikörper bis zu 0,7 µmol beträgt.

10. Standard für einen Blutgerinnungstest, umfassend normales menschliches Plasma, behandelt oder gemischt mit dem Antikörper nach einem der Ansprüche 1 bis 5.

11. Teststandard nach Anspruch 10 zur Verwendung in Blutgerinnungstests, ausgewählt aus der Gruppe, bestehend aus dem Test auf die aktivierte partielle Thromboplastinzeit (APTT), der Kaolin-Gerinnungszeit (KCT), dem Gewebsthromboplastin-Inhibitionstest (TTTT), der Zeit von verdünntem Russell-Schlangengift (dRVVT = dilute Russells's viper venom time), Taipan-Gift und dem Textarintest.

12. Verfahren zum Erhalt eines Antikörpers, gerichtet gegen den F1-Bereich des Prothrombins, der die Wirkung eines Lupus-Anticoagulans (LA) in einem *In-Vitro*-Blutgerinnungstest nachahmt, wobei das Verfahren umfasst:
das Immunisieren eines nicht menschlichen Tieres mit Prothrombin oder einem N-terminalen Peptid, abgeleitet von menschlichem Prothrombin;
das Testen auf die Produktion eines Antikörpers durch das Tier, der den F1.2-Bereich des Prothrombins binden kann, jedoch den F2-Bereich des Prothrombins nicht binden kann und welcher Antikörper die Wirkungen eines Lupus-Anticoagulans (LA) in einem *In*-*Vitro*-Blutgerinnungstest nachahmt, und
Isolieren des Antikörpers.

## Revendications

1. Anticorps isolé destiné à être utilisé dans une analyse de coagulation sanguine *in vitro*, anticorps
**caractérisé en ce que** :
il est dirigé contre la région F1 de la prothrombine ;
il est capable de se lier à la région F1.2 de la prothrombine mais il n'est pas capable de se lier à la région F2 de la prothrombine ; et
il est capable de mimer l'effet d'un anticoagulant du lupus (LA) dans une analyse de coagulation sanguine *in vitro*.

2. Anticorps suivant la revendication 1, qui est un anticorps monoclonal.

3. Anticorps suivant la revendication 2, obtenu à partir d'une souris après immunisation avec de la prothrombine humaine et choisi pour son aptitude à mimer l'effet d'un anticoagulant du lupus (LA) dans une analyse de coagulation sanguine *in vitro*.

4. Anticorps suivant la revendication 3, dans lequel la souris est immunisée avec un peptide N-terminal dérivé de la prothrombine humaine.

5. Anticorps suivant l'une quelconque des revendications 1 à 4, dans lequel l'analyse de coagulation sanguine est choisie dans le groupe consistant en le test de céphaline-kaolin (APTT), le test de temps de coagulation en présence de kaolin (KCT), le test d'inhibition de thromboplastine tissulaire (TTIT), le test de temps en présence de venin dilué de vipère de Russel (DRVVT), le test de temps en présence de venin de Taipan et le test Textarin.

6. Utilisation d'un anticorps isolé suivant l'une quelconque des revendications 1 à 5 dans une analyse de coagulation sanguine *in vitro*.

7. Utilisation suivant la revendication 6, dans laquelle l'analyse de coagulation sanguine est choisie dans le groupe consistant en le test de céphaline-kaolin (APTT), le test de temps de coagulation en présence de kaolin (KCT), le test d'inhibition de thromboplastine tissulaire (TTIT), le test de temps en présence de venin dilué de vipère de Rusael (DRVVT), le test de temps en présence de venin de Taipan et le test Textarin.

8. Utilisation suivant la revendication 6 ou 7, dans laquelle la quantité d'anticorps est comprise entre 0,01 et 10 µmol.

9. Utilisation suivant la revendication 8, dans laquelle la quantité d'anticorps va jusqu'à 0,7 µmol.

10. Echantillon de référence de test de coagulation sanguine, comprenant du plasma humain normal, traité ou mélangé à l'anticorps, suivant l'une quelconque des revendications 1 à 5.

11. Echantillon de référence de test suivant la revendication 10, destiné à être utilisé dans des tests de coagulation sanguine choisis dans le groupe consistant en le test de céphaline-kaolin (APTT), le test de temps de coagulation en présence de kaolin (KCT), le test d'inhibition de thromboplastine tissulaire (TTIT), le test de temps en présence de venin dilué de vipère de Russel (DRVVT), le test de temps en présence de venin de Taipan et le test Textarin.

12. Procédé pour obtenir un anticorps dirigé contre la région F1 de la prothrombine, qui mime l'effet d'un anticoagulant du lupus (LA) dans un test de coagulation sanguine *in vitro*, procédé comprenant les étapes consistant à :
immuniser un animal non humain avec de la prothrombine ou un peptide N-terminal dérivé de la prothrombine humaine ;
tester la production par l'animal d'un anticorps qui est capable de se lier à la région F1.2 de la prothrombine mais qui n'est pas capable de se lier à la région F2 de la prothrombine, anticorps qui mime l'effet d'un anticoagulant du lupus (LA) dans un test de coagulation sanguine *in vitro* ; et
isoler l'anticorps.


### SEQUENCE LISTING

(1) GENERAL INFORMATION:
   (i) APPLICANT:
      (A) NAME: Gradipore Limited
      (B) STREET: 35-105 Delhi Road
      (C) CITY: North Ryde
      (D) STATE: New South Wales
      (E) COUNTRY: Australia
      (F) POSTAL CODE (ZIP): 2113

      (A) NAME: Dade Behring Marburg GmbH
      (B) STREET: PO Box 1149
      (C) CITY: Marburg
      (E) COUNTRY: Germany
      (F) POSTAL CODE (ZIP): D-35001
   (ii) TITLE OF INVENTION: Lupus Anticoagulant
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO: 1:
   Cys Gly Ser Asp Arg Ala Ile Glu Gly Arg
   1               5                   10